# EUROPÄISCHE PATENTANMELDUNG

(11) **EP 4 477 181 A1**
(43) Veröffentlichungstag der Anmeldung: **18.12.2024**
(21) Anmeldenummer: 23179521.2
(22) Anmeldetag: 15.06.2023
(51) Int. Cl.: A61B 90/13, A61B 10/02, A61B 17/34

(54) **BIOPSIEEINRICHTUNG, UNTERSTÜTZUNGSVERFAHREN UND BIOPSIEGERÄT**

(71) Anmelder: Siemens Healthineers AG, 91301 Forchheim (DE)
(72) Erfinder: Haeßler, Christian, 91088 Bubenreuth (DE)
(74) Vertreter: Siemens Healthineers Patent Attorneys

(57) **Zusammenfassung**

Die Erfindung betrifft eine Biopsieeinrichtung (1) zum Durchführen einer Biopsie an einer weiblichen Brust (10), aufweisend:
- eine Bildgebungseinrichtung (2) zur Tomosynthese der Brust (10) mit einer Kompressionseinrichtung (43) zur Fixierung der Brust (10),
- ein handgehaltenes Biopsiegerät (3) mit einer Biopsienadel, wobei das Biopsiegerät (3) einen Lagesensor (12) zur Messung von die Orientierung der Biopsienadel (8) anzeigenden Lageinformationen aufweist,
- eine mit der Bildgebungseinrichtung (2) registrierte Markierungseinrichtung (6) zur Markierung eines Eintrittspunktes für die Biopsienadel (8) auf der in der Kompressionseinrichtung (43) fixierten Brust (10) gemäß einer auf Bilddaten der Bildgebungseinrichtung (2) basierenden Planungsinformation für eine durchzuführende Biopsie,
- eine Ausgabeeinrichtung (5) zur Ausgabe einer Führungsinformation, die die relative Orientierung der Biopsienadel (8) gemäß der Lageinformation zu einer Sollorientierung (41) gemäß der Planungsinformation anzeigt, für eine die Biopsie durchführende Person, und
- eine Steuereinrichtung (4) zur Steuerung der Biopsieeinrichtung (1) gemäß der Planungsinformation.

## Beschreibung

Die Erfindung betrifft eine Biopsieeinrichtung zum Durchführen einer Biopsie an einer weiblichen Brust, die eine Bildgebungseinrichtung zur Tomosynthese der Brust mit einer Kompressionseinrichtung zur Fixierung der Brust aufweist. Daneben betrifft die Erfindung ein Verfahren zur Unterstützung einer eine Biopsie mit einer Biopsieeinrichtung durchführenden Person und ein Biopsiegerät.

Biopsien an der weiblichen Brust (Mamma) oder am sonstigen Stellen des menschlichen Körpers werden üblicherweise durchgeführt, um anatomische Auffälligkeiten, insbesondere Gewebeveränderungen, genauer untersuchen und in der Folge diagnostizieren zu können. Dies liegt darin begründet, dass die medizinische Bildgebung, beispielsweise Brust-Tomosynthese, häufig nicht ausreichend ist, um eine abschließende Diagnose stellen zu können. Bei einer Biopsie wird eine Biopsienadel als medizinisches Instrument genutzt, um möglichst minimalinvasiv an die interessierende Stelle gelangen zu können und dort eine Gewebeprobe zu entnehmen. Beispielsweise ist hierzu die Methodik der sogenannten Stanzbiopsie bekannt.

In der Biopsie ist es wesentlich, die interessierende Stelle innerhalb der Patientin möglichst exakt zu treffen, das bedeutet, die Biopsienadel muss im dreidimensionalen Raum mit hinreichender Positioniergenauigkeit positioniert werden. Hierfür weisen Biopsieeinrichtungen üblicherweise Bildgebungseinrichtungen, üblicherweise Röntgeneinrichtungen, auf. In vielen Fällen, um an der Biopsieeinrichtung auch ein Screening durchführen zu können, handelt es sich bei der Bildgebungseinrichtung um eine Tomosyntheseeinrichtung. An Biopsieeinrichtungen werden dabei eine Vielzahl von Anforderungen gestellt. Hinsichtlich der Bildgebung sollen Bildartefakte bei der Röntgenaufnahme durch die Biopsienadel und Komponenten einer die Biopsienadel beinhaltenden Biopsieeinheit möglichst vermieden werden, sowohl bei zweidimensionalen Röntgenaufnahmen als auch bei dreidimensionalen Röntgenaufnahmen, insbesondere Tomosynthese-Aufnahmen. Das Biopsiegerät und sonstige patientennah zu positionierende Komponenten sollen möglichst geringen Bauraum auf und an dem Objekttisch in Anspruch nehmen, um den Zugang zur Patientin und zum Untersuchungsbereich nicht einzuschränken. Die Behandlungsdauer soll möglichst kurz sein. Beispielsweise bedeutet dies, dass motorische Positionierungsfahrten von kurzer Dauer sein sollten. Schließlich sind ein geringes Gewicht und geringe Kosten wünschenswert.

Im Stand der Technik wurden bereits Ausgestaltungen von Biopsieeinrichtungen vorgeschlagen, bei denen eine Biopsieeinheit, die Aktoren zum Positionieren und Bewegen der Biopsienadel aufweisen, am Objekttisch und/oder am Stativ der Bildgebungseinrichtung befestigt wird. Die gesamte Biopsieeinheit ist dabei mit dem Koordinatensystem der Bildgebungseinrichtung registriert, sodass eine mithilfe von Bilddaten der Bildgebungseinrichtung erhaltene Planungsinformation unmittelbar in eine Ansteuerung der insbesondere als Elektromotoren ausgebildeten Aktoren umgesetzt werden kann, um vollständig automatisch eine Gewebeentnahme durchzuführen.

Eine derartige Ausgestaltung ist jedoch dahingehend nachteilhaft, dass derartige, fest installierte bzw. fest installierbare Biopsieeinheiten aufwendig sind, einen großen Bauraum einnehmen und auf verlässliche und robuste Weise hochkomplexe derart umgesetzt werden müssen, dass die interessierende Stelle hinreichend genau getroffen wird. Darüber hinaus ist die Akzeptanz seitens der Patientinnen teilweise eingeschränkt, da die Biopsie vollautomatisch ohne Mitwirkung eines Vertrauen erweckenden Menschen durchgeführt wird.

Der Erfindung liegt daher die Aufgabe zugrunde, eine Biopsieeinrichtung anzugeben, die trotz kostengünstiger, bauraumsparender Realisierbarkeit auf einfache Weise eine hochgenaue Biopsie erlaubt.

Zur Lösung dieser Aufgabe sind erfindungsgemäß eine Biopsieeinrichtung, ein Verfahren und ein Biopsiegerät gemäß den unabhängigen Patentansprüchen vorgesehen. Vorteilhafte Ausgestaltungen ergeben sich aus den Unteransprüchen.

Eine Biopsieeinrichtung zum Durchführen einer Biopsie an einer weiblichen Brust weist erfindungsgemäß auf:
- eine Bildgebungseinrichtung zur Tomosynthese der Brust mit einer Kompressionseinrichtung zur Fixierung der Brust,
- ein handgehaltenes Biopsiegerät mit einer Biopsienadel, wobei das Biopsiegerät einen Lagesensor zur Messung von die Orientierung der Biopsienadel anzeigenden Lageinformationen aufweist,
- eine mit der Bildgebungseinrichtung registrierte Markierungseinrichtung zur Markierung eines Eintrittspunktes für die Biopsienadel auf der in der Kompressionseinrichtung fixierten Brust gemäß einer auf Bilddaten der Bildgebungseinrichtung basierenden Planungsinformation für eine durchzuführende Biopsie,
- eine Ausgabeeinrichtung zur Ausgabe einer Führungsinformation, die die relative Orientierung der Nadel gemäß der Lageinformation zu einer Sollorientierung gemäß der Planungsinformation anzeigt, für eine die Biopsie durchführende Person, und
- eine Steuereinrichtung zur Steuerung der Biopsieeinrichtung gemäß der Planungsinformation.

Dabei ist der Lagesensor zur Aufnahme von Sensordaten, die die Orientierung der Biopsienadel in drei Dimensionen beschreiben, ausgebildet. Der Lagesensor kann insbesondere ein gyroskopischer Sensor sein, aber auch anderweitig ausgestaltet sein.

Erfindungsgemäß wird mithin vorgeschlagen, ein handgehaltenes Biopsiegerät, welches die Biopsienadel beinhaltet und einen Lagesensor aufweist, mit einer Markierungseinrichtung derart zu kombinieren, dass auf platzsparende und kostengünstige Weise dennoch eine hochgenaue Positionierung der Biopsienadel zur Entnahme der Gewebeprobe auf Basis einer Planungsinformation, die auf Bilddaten der Bildgebungseinrichtung beruht, möglich ist. Hierbei wird eine Ausgabeeinrichtung verwendet, die die Person, die die Biopsie durchführt, derart informiert, dass die Biopsienadel gemäß der Sollorientierung ausgerichtet werden kann. Über den Soll-Eintrittspunkt wird mittels der Markierungseinrichtung informiert.

Auf diese Weise kann die Biopsieeinrichtung äußerst kostengünstig und handlich realisiert werden, da keinerlei Motoren, Leistungselektronik oder bewegliche Achsen- und/oder Präzisionsführungen benötigt werden. Das Biopsiegerät kann mit sehr geringem Gewicht umgesetzt werden und daher durch die die Biopsie durchführende Person gut geführt werden, insbesondere zur Einstellung der Sollorientierung. Nachdem das handgehaltene Biopsiegerät vom Objekttisch entfernt werden kann und insbesondere nicht, wie eine hochkomplexe, schwere Biopsieeinheit des Standes der Technik, am Objekttisch oder dem Stativ befestigt werden muss, existieren gegenüber einer Bildgebungseinrichtung alleine kaum Bauraumeinschränkungen und der freie Zugang zur Patientin wird vollständig erhalten. Zugleich liegt ein hohes Sicherheitsgefühl für die Patientinnen vor, da der Eingriff handgeführt durch einen Menschen durchgeführt wird.

Es ist eine schnelle Probenentnahme, wie beispielsweise auch bei der ultraschallgeführten Biopsie, möglich. Nachdem keine Biopsieeinheit installiert werden muss, ist kein Umrüstaufwand zwischen dem Screening und der Biopsie notwendig. Hierbei sei angemerkt, dass der erfindungsgemäße Vorschlag auf einfache Art und Weise auch eine Nachrüstung bestehender Bildgebungseinrichtungen, insbesondere von Mammografieeinrichtungen, zur Bildung einer Biopsieeinrichtung erlaubt, nachdem letztlich lediglich die Markierungseinrichtung und das handgehaltene Biopsiegerät hinzugefügt werden müssen. Eine Steuereinrichtung und ein Display oder dergleichen sind meist bereits vorhanden.

Im konkreten Betrieb wird immer dann, wenn eine Planungsinformation vorliegt, die beispielsweise einen gewünschten Pfad der Biopsienadel beschreiben kann, der Eintrittspunkt an die Markierungseinrichtung übertragen bzw. die Markierungseinrichtung gemäß dem Eintrittspunkt zu dessen Markierung angesteuert. Anders gesagt kann die Steuereinrichtung insgesamt zum Übertragen des Eintrittspunktes an die Markierungseinrichtung und/oder zur Ansteuerung der Markierungseinrichtung gemäß dem Eintrittspunkt ausgebildet sein.

Insbesondere kann die Markierungseinrichtung einen Markierungslaser aufweisen. Es ist also denkbar, den Eintrittspunkt durch Licht auf der Oberfläche der Patientin zu markieren. Hierzu ist die Verwendung von Laserlicht aufgrund dessen guter Erkennbarkeit bevorzugt, wobei jedoch auch andere Arten von Projektionseinrichtungen verwendet werden können. Eine beispielhaft einsetzbare Markierungseinrichtung wurde beispielsweise in der Veröffentlichung "Integration eines Projektors zur Einblendung von Positionier- und Untersuchungshilfen" in Prior Art Journal 2020 #05 - Pages: 119-122 - ISBN: 978-3-947591-33-6; Volume No.: 99, beschrieben.

Zweckmäßigerweise kann die Markierungseinrichtung an der Bildgebungseinrichtung angeordnet sein. Durch eine Anordnung der Markierungseinrichtung an der Bildgebungseinrichtung ist eine Registrierung auf besonders einfache Art und Weise gegeben. Konkret und besonders bevorzugt kann die Markierungseinrichtung dabei an einer den Röntgenstrahler der Bildgebungseinrichtung umfassenden Strahlereinheit angeordnet sein bzw. in diese integriert sein. Bei Mammografieeinrichtungen und bekannten Biopsieeinrichtungen ist es bekannt, den Röntgendetektor mit einer Abdeckung selbst als Objekttisch einzusetzen, wobei die Brust mittels einer zusätzlichen, oberen Kompressionsplatte (auch als Kompressionspaddle bezeichnet) komprimiert und in der Position gehalten werden kann. Der Röntgenstrahler ist in der Strahlereinheit, bei möglicher Tomosynthese beweglich, oberhalb des Objekttisches und der Kompressionsplatte angeordnet, von wo sich hervorragend die Soll-Eintrittsposition markieren lässt.

In diesem Zusammenhang ist es auch besonders vorteilhaft, wenn die Kompressionseinrichtung die obere, insbesondere von dem Röntgendetektor der Bildgebungseinrichtung abgewandt die Brust komprimierende Kompressionsplatte aufweist, wobei die Kompressionsplatte wenigstens eine Durchgangsöffnung zum Durchführen der Biopsienadel aufweist. Wie bereits erwähnt, liegt dabei die Brust unten bevorzugt auf einer Detektorabdeckung des Röntgendetektors auf, die den Objekttisch bildet. Es wird also eine obere Kompressionsplatte verwendet, die Zugang zur Brust zumindest in durch die Durchgangsöffnungen, insbesondere wenigstens ein Fenster, definierten Bereichen gestattet. Diese Durchgangsöffnungen können zum einen genutzt werden, um den Eintrittspunkt, bevorzugt, wie erwähnt, von oben, zu projizieren, zum anderen auch, um mit der Biopsienadel des Biopsiegeräts Zugang zu erhalten. In einer konkreten Ausgestaltung kann vorgesehen sein, dass die Kompressionsplatte mehrere, matrixartig angeordnete Durchgangsöffnungen aufweist. Eine solche Kompressionsplatte kann auch als "alphanumerische Kompressionsplatte" bezeichnet werden, nachdem eine Art "Schachbrettmuster" für die Durchgangsöffnungen vorgesehen sein kann und die einzelnen Durchgangsöffnungen mit einem Buchstaben (für eine Richtung) und einer Zahl (für die andere Richtung) identifiziert werden können.

In Weiterbildung der Erfindung kann vorgesehen sein, dass sie eine, insbesondere an der Bildgebungseinrichtung angeordnete, Halterung für das Biopsiegerät aufweist. Beispielsweise kann die Halterung seitlich an der Bildgebungseinrichtung, insbesondere an einem Stativ der Bildgebungseinrichtung, angeordnet sein, jedoch sind auch andere Anordnungspositionen denkbar. In der Halterung kann das Biopsiegerät abgelegt werden, solange es nicht benötigt wird, beispielsweise, wenn die Bildgebungseinrichtung zum Screening verwendet werden soll. Zweckmäßigerweise kann die Halterung verschiedenen weiteren Funktionen dienen.

So kann konkret vorgesehen sein, dass die Halterung eine vorbestimmte Kalibrierungsorientierung des Biopsiegeräts zum Kalibrieren des Lagesensors herstellend ausgebildet ist. Dies kann beispielsweise anhand einer Formgebung und/oder anhand entsprechend gewählter Stützflächen erreicht werden. Insbesondere kann die Halterung so ausgestaltet sein, dass das Biopsiegerät nur in der bestimmten Kalibrierungsorientierung passgenau in sie eingesetzt werden kann. Immer also dann, wenn das Biopsiegerät in die Halterung eingesetzt ist, kann der Lagesensor neu kalibriert werden, so dass die Lageinformation immer klar definiert in einer mit der Bildgebungseinrichtung registrierten Art ermittelt werden kann.

Die Halterung kann ferner eine elektrische Schnittstelle zum Aufladen eines elektrischen Energiespeichers des Biopsiegeräts aufweisen. Hierbei kann die Schnittstelle beispielsweise halterungsseitige Anschlussmittel umfassen, die mit biopsiegeräteseitigen Anschlussmitteln zusammenwirken. Insbesondere wird beim Einsetzen des Biopsiegeräts in die Halterung unmittelbar auch die elektrische Verbindung zum Aufladen des elektrischen Energiespeichers hergestellt. Die Halterung kann in diesem Sinne auch als eine Art "Ladeschale" verstanden werden.

Auch allgemein gesprochen ist es zweckmäßig, wenn das Biopsiegerät kabellos ausgestaltet ist, mithin keinerlei Anschlusskabel benötigt, die bei der Biopsie stören und/oder behindern könnten. Konkret kann vorgesehen sein, dass, insbesondere zusätzlich zu dem Energiespeicher, das Biopsiegerät eine Kommunikationseinrichtung zur drahtlosen Kommunikation mit der Steuereinrichtung aufweist. Beispielsweise kann die Kommunikationseinrichtung eine Bluetooth-Einrichtung sein.

Vorzugsweise kann das Biopsiegerät eine Steuereinheit aufweisen, die über die Kommunikationseinrichtung mit der Steuereinrichtung kommunizieren kann, insbesondere bidirektional. Die Steuereinheit steuert den Betrieb des Biopsiegeräts. Sie kann insbesondere ausgebildet sein, aus den Sensordaten des Lagesensors die Lageinformation zu ermitteln und
- die Lageinformation oder eine daraus abgeleitete Information mittels der Kommunikationseinrichtung an die (biopsiegerätexterne) Steuereinrichtung zu übermitteln und/oder
- eine Abweichung der Orientierung der Biopsienadel von der Sollorientierung, die über die Kommunikationseinrichtung erhalten wurde, zu ermitteln.

Hierbei ist es insbesondere zweckmäßig, wenn sowohl die Steuereinrichtung über die aktuelle Lageinformation informiert ist als auch die Steuereinheit die Sollorientierung kennt. Denn dann ist es, wie im Folgenden noch genauer dargelegt werden wird, denkbar, die Ausgabeeinrichtung letztlich verteilt umzusetzen, um auf vielfältige Art und Weise die den Eingriff durchführende Person mit Unterstützungshinweisen als Führungsinformation zu unterstützen.

In einer besonders bevorzugten Ausgestaltung der vorliegenden Erfindung ist vorgesehen, dass das Biopsiegerät ein Ausgabemittel der Ausgabeeinrichtung aufweist, welches zur Ausgabe eines Unterstützungshinweises in Abhängigkeit der relativen Orientierung der Biopsienadel gemäß der Lageinformation zu der Sollorientierung ausgebildet ist. Ist das Ausgabemittel unmittelbar am Biopsiegerät vorgesehen, muss die die Biopsie durchführende Person ihre Aufmerksamkeit nicht von dem Biopsiegerät und dem Eingriffsbereich, insbesondere auch dem markierten Eintrittspunkt, abwenden und kann sich daher auf vereinfachte Weise vollständig auf den Eingriff konzentrieren. Dabei kann die Ansteuerung des Ausgabemittels zweckmäßig durch die oben beschriebene Steuereinheit direkt erfolgen, wenn die Abweichung von der Sollorientierung, wie oben beschrieben, biopsiegeräteintern ermittelt wird. In diesem Fall muss also lediglich dem Biopsiegerät die Sollorientierung von der Steuereinrichtung bereitgestellt werden, wozu die Kommunikationseinrichtung genutzt werden kann, damit das Biopsiegerät autark durch entsprechende Ausgaben über das Ausgabemittel die die Biopsie durchführende Person zur Einnahme der Sollorientierung führen kann, während der Eintrittspunkt extern durch die mit der Bildgebungseinrichtung registrierte Markierungseinrichtung angezeigt wird.

Besonders bevorzugt kann dabei das Ausgabemittel ein haptisches Ausgabemittel sein. Konkret kann es sich bei dem haptisches Ausgabemittel um einen Vibrationsmotor handeln. Auf diese Weise kann die den Eingriff durchführende Person den Blick weiterhin auf den tatsächlichen Biopsiebereich, insbesondere den Eintrittspunkt und die Biopsienadel, richten, nachdem sich das Ausgabemittel an andere Sinne richtet. Da das Biopsiegerät handgehalten ist, sind Vibrationen gut spürbar. Zusätzlich oder alternativ kann das Ausgabemittel auch ein akustisches Ausgabemittel sein. Auch ist ein optisches Ausgabemittel denkbar, zumindest alleinstehend jedoch weniger bevorzugt. Dabei ist es grundsätzlich denkbar, Feedback immer dann zu geben, wenn sich die Biopsienadel in der Sollorientierung befindet. Bevorzugt ist es jedoch, das Feedback über das Ausgabemittel, insbesondere das haptische Ausgabemittel, zu nutzen, um die die Biopsie durchführende Person schrittweise zu der Sollorientierung zu führen.

In einer konkreten, vorteilhaften Ausgestaltung kann daher das Ausgabemittel gemäß einem sich über die Zeit verkleinernden, einzustellenden Zielbereich um die Sollorientierung zur schrittweisen Führung der die Biopsie durchführenden Person zu der Sollorientierung ansteuerbar sein. Insbesondere kann also die Steuereinheit ausgebildet sein, diese Ansteuerung durchzuführen. Mit anderen Worten wird vorgeschlagen, von einem großen "Fangbereich" zu einem kleineren "Fangbereich" überzugehen. Hierbei kann konkret vorgesehen sein, dass das Ausgabemittel bei Verlassen des Zielbereichs aktiviert wird. Das bedeutet, immer dann, wenn die die Biopsie durchführende Person droht, den Zielbereich zu verlassen, wird sie durch entsprechendes Feedback wieder in diesen zurückgeleitet. Mit der zunehmenden Verkleinerung des Zielbereichs über die Zeit wird somit immer genauer die Sollorientierung eingenommen, indem erst zu einem groben Zielbereich geführt wird, welcher dann zunehmend verengt wird. Somit ist eine besonders intuitive Zielführung zur Sollorientierung gegeben.

Es sind auch andere Ansätze denkbar, das Ausgabemittel konkret zu nutzen, beispielsweise im Fall eines akustischen Ausgabemittels den Benutzer nach Art einer "Einparkhilfe" zu der Sollorientierung zuführen, indem bei größerem Abstand zur Sollorientierung zeitlich beabstandete Töne ausgegeben werden, deren zeitlicher Abstand zueinander kleiner wird, bis ein Durchgangston bei Erreichen der Sollorientierung vorliegt.

Alternativ oder vorzugsweise zusätzlich kann vorgesehen sein, dass die Ausgabeeinrichtung ein Display umfasst, wobei die Steuereinrichtung zur Ansteuerung des Displays zur Ausgabe einer die relative Orientierung der Nadel gemäß der Lageinformation zu der Sollorientierung visualisierenden Darstellung ansteuert. Die Darstellung bildet dann also die Führungsinformation. Hierbei kann beispielsweise ein Display verwendet werden, das durch die Bildgebungseinrichtung auch eingesetzt wird, um Bilddaten darzustellen und dergleichen. Bei dem Display kann es sich auch um einen Touchscreen handeln, der zusätzlich als Eingabeeinrichtung nutzbar ist. Bevorzugt ist das Display in einem Sichtbereich der die Biopsie durchführenden Person angeordnet oder anordenbar, beispielsweise mittels einer verstellbaren Halteeinrichtung, wie beispielsweise einem Haltearm.

Das Display wird hierbei bevorzugt von der Steuereinrichtung direkt gesteuert, welche mittels der Kommunikationseinrichtung die Lageinformation von dem Biopsiegerät erhält. Die graphische Darstellung kann insbesondere eine abstrahierte und/oder schematische Darstellung der Biopsienadel in der aktuellen, durch die Lageinformation beschriebenen Orientierung im Vergleich zu der durch ein zusätzliches Element dargestellten Sollorientierung enthalten. Diese Darstellung kann beispielsweise als Überlagerung von Bilddaten, insbesondere solchen Bilddaten, die der Planungsinformation zugrunde liegen, ermittelt werden. Auch bezüglich des Hintergrunds ist jedoch eine Abstraktion möglich, insbesondere, nachdem im Allgemeinen die aktuelle tatsächliche räumliche Position der Biopsienadel - im Gegensatz zu ihrer Orientierung - nicht bekannt ist und somit eine Verwirrung vermieden werden kann.

Die Biopsieeinrichtung kann ferner eine Planungseinrichtung zur wenigstens teilweise automatischen Ermittlung der Planungsinformation aus Bilddaten der Bildgebungseinrichtung aufweisen. Dabei können beispielsweise mit der Bildgebungseinrichtung zwei- oder dreidimensionale Scout-Scans aufgenommen werden, die wenigstens teilweise automatisch durch die Planungseinrichtung, welche beispielsweise eine Workstation umfassen kann, ausgewertet werden können. Beispielsweise kann auf einer Anzeigeeinrichtung der Workstation ein Planungs-Benutzerinterface dargestellt werden, über das ein Benutzer manuell in die Planung eingreifen kann, beispielsweise die interessierende Stelle markieren kann und dergleichen. Planungsvorgänge sowie allgemein Vorgehensweisen zur Ermittlung der Planungsinformation sind im Stand der Technik bereits weitgehend bekannt und sollen hier nicht im Detail dargelegt werden.

Vorzugsweise kann die Biopsienadel, insbesondere entlang des Nadelschafts, Markierungen zur Anzeige einer Eindringtiefe in eine Patientin aufweisen. So können in regelmäßigen Abständen entlang des Nadelschafts der Biopsienadel Markierungen vorgesehen sein, sodass die aktuelle Eindringtiefe durch die die Biopsie durchführende Person leicht abgelesen werden kann. Die Soll-Eindringtiefe gemäß der Planungsinformation kann beispielsweise mittels der Ausgabeeinrichtung, insbesondere dem Display, zur Referenz ausgegeben werden. Neben den Parametern Eintrittspunkt und Sollorientierung ist es mittels solcher Markierungen auch möglich, die Soll-Eindringtiefe zu überwachen und zu prüfen. Dabei sei angemerkt, dass eine aktuelle Eindringtiefe alternativ oder zusätzlich, falls entsprechende Erfassungsmittel in dem Biopsiegerät vorgesehen sind, auch an einem oder dem Ausgabemittel des Biopsiegeräts ausgegeben werden kann. Auch ist es grundsätzlich denkbar, in dem Biopsiegerät einen Aktor zum automatischen Ausfahren der Biopsienadel vorzusehen, was jedoch weniger bevorzugt ist, da Aktoren, wie angesprochen, bevorzugt vermieden werden, um das Biopsiegerät handhabbar und leicht zu halten.

Neben der Biopsieeinrichtung betrifft die vorliegende Erfindung auch ein Verfahren zur Unterstützung einer eine Biopsie mit einer erfindungsgemäßen Biopsieeinrichtung durchführenden Person, wobei die Ausgabeeinrichtung zur Ausgabe einer Führungsinformation, die die relative Orientierung der Nadel gemäß der Lageinformation zu einer Sollorientierung gemäß der Planungsinformation anzeigt, angesteuert wird. Sämtliche Ausführungen zur erfindungsgemäßen Biopsieeinrichtung, insbesondere hinsichtlich der Kommunikation, der Steuerung durch die Steuereinrichtung und die Steuereinheit sowie der konkreten Ausgestaltung der Ausgabeeinrichtung, lassen sich analog auf das erfindungsgemäße Verfahren übertragen, mit welchem mithin ebenso die bereits genannten Vorteile erhalten werden können.

Schließlich betrifft die Erfindung auch ein handgehaltenes Biopsiegerät für eine erfindungsgemäße Biopsieeinrichtung, aufweisend eine Biopsienadel und einen Lagesensor zur Messung von die Orientierung der Biopsienadel anzeigenden Lageinformationen. Auch für das Biopsiegerät gelten die Ausführungen zur Biopsieeinrichtung und zum Verfahren, soweit anwendbar, analog fort. Insbesondere kann das Biopsiegerät daher auch die Steuereinheit, den elektrischen Energiespeicher, die Kommunikationseinrichtung und/oder Anschlussmittel zum Aufladen des elektrischen Energiespeichers aufweisen. Besonders bevorzugt jedoch weist das Biopsiegerät ein Ausgabemittel auf, das, wie oben beschrieben, insbesondere durch die Steuereinheit ansteuerbar ist, um die relative Orientierung zu bzw. Abweichung von einer, insbesondere über die Kommunikationseinrichtung erhaltenen, Sollorientierung anzuzeigen, beispielsweise gemäß einem zeitlich kleiner werdenden Zielbereich um die Sollorientierung.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Skizze funktionaler Komponenten einer erfindungsgemäßen Biopsieeinrichtung,
- Fig. 2: eine perspektivische Ansicht eines Biopsiegeräts,
- Fig. 3: eine Ansicht eines Teils einer Bildgebungseinrichtung,
- Fig. 4: eine Kompressionsplatte,
- Fig. 5: einen Graphen zur Erläuterung der Ansteuerung eines Ausgabemittels, und
- Fig. 6: eine mögliche visuelle Darstellung auf einem Display.

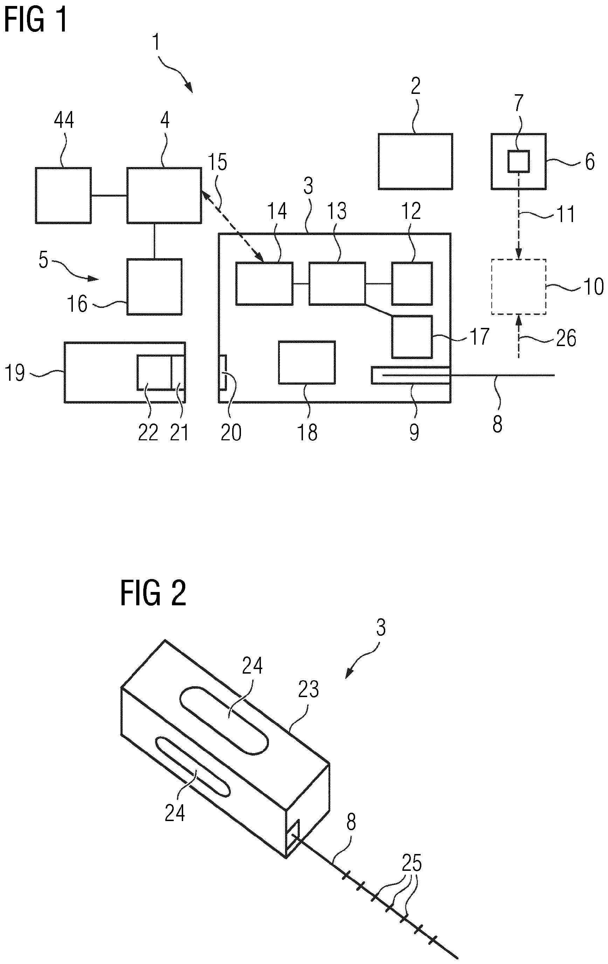

Fig. 1 zeigt den funktionalen Aufbau einer erfindungsgemäßen Biopsieeinrichtung 1. Die Biopsieeinrichtung 1 umfasst als hauptsächliche Komponenten eine hier nicht näher aufgeschlüsselte Bildgebungseinrichtung 2, ein handgehaltenes, kabelloses Biopsiegerät 3, eine Steuereinrichtung 4, eine vorliegend in mehrere Komponenten aufgeteilte Ausgabeeinrichtung 5 und eine Markierungseinrichtung 6, die vorliegend an der Bildgebungseinrichtung 2 befestigt oder in dieser integriert ist. Zusätzlich umfasst die Biopsieeinrichtung 1 vorliegend noch eine Planungseinrichtung 44, die beispielsweise als eine Workstation ausgebildet sein kann.

Die Planungseinrichtung 44 ist ausgebildet, wenigstens teilweise automatisch basierend auf Bilddaten der Bildgebungseinrichtung 2, vorliegend eine Röntgeneinrichtung zur Tomosynthese der weiblichen Brust, eine Planung durchzuführen, an der auch ein Benutzer beteiligt sein kann. Das Ergebnis der Planung ist eine Planungsinformation, die insbesondere angibt, wie eine Biopsienadel 8 des Biopsiegeräts 3, die dort mittels einer Nadelhalterung 9 befestigt ist, zu einer interessierenden Stelle, wo eine Gewebeprobe entnommen werden soll, innerhalb der aktuell zu behandelnden, hier schematisch angedeuteten Brust 10 gelangen soll. Die Planungsinformation beschreibt also insbesondere einen Soll-Eintrittspunkt, eine Sollorientierung und auch eine Soll-Eindringtiefe der Biopsienadel 8. Diese Planungsinformation wird der Steuereinrichtung 4 bereitgestellt.

Die Steuereinrichtung 4 gibt vorliegend den Eintrittspunkt an die Markierungseinrichtung 6 weiter, welche einen Markierungslaser 7 aufweist und mit der Bildgebungseinrichtung 2, auf deren Koordinatensystem sich auch die Planungsinformation bezieht, registriert ist. Daher kann die Markierungseinrichtung 6 den Eintrittspunkt gemäß dem gestrichelten Pfeil 11 für eine die Biopsie durchführende Person auf die Brust 10 hochgenau projizieren.

Um auch die Sollorientierung korrekt einstellen zu können, weist das Biopsiegerät 3 einen Lagesensor 12, beispielsweise einen gyroskopischen Sensor, auf, der die aktuelle Orientierung des Biopsiegeräts 3 in allen drei Raumrichtungen vermessen kann. Die Sensordaten des Lagesensors 12 werden durch eine Steuereinheit 13 des Biopsiegeräts 3 zu einer Lageinformation ausgewertet. Über eine Kommunikationseinrichtung 14 kann das Biopsiegerät 3 drahtlos mit der Steuereinrichtung 4 kommunizieren, wie durch den Pfeil 15 angedeutet ist. Vorliegend ist die Kommunikationseinrichtung 14 als ein Bluetooth-Modul ausgebildet und die Steuereinrichtung 4 Bluetooth-fähig. Von der Steuereinrichtung 4 erhält die Steuereinheit 13 über die Kommunikationseinrichtung 14 die Sollorientierung bereitgestellt, sodass sie eine Abweichung der aktuellen Orientierung des Biopsiegeräts 3 und somit der Biopsienadel 8 von der Sollorientierung feststellen kann. Gleichzeitig kann die Steuereinheit 13 der Steuereinrichtung 4 über die Kommunikationseinrichtung 14 die Lageinformation bereitstellen.

Die Steuereinrichtung 4 sowie die Steuereinheit 13 nutzen unterschiedliche Komponenten der Ausgabeeinrichtung 5, um der die Biopsie durchführenden, das Biopsiegerät 3 in der Hand haltenden Person als Führungsinformation Unterstützungshinweise, die sie zur Einnahme der Sollorientierung durch die Biopsienadel 8 führen, zu geben.

Konkret weist zunächst die Biopsieeinrichtung 1 im Allgemeinen ein Display 16 als Teil der Ausgabeeinrichtung 5 auf, auf dem eine grafische Darstellung visuell eine potentielle Abweichung von der Sollorientierung anzeigt. Eine derartige Darstellung kann seitens der Steuereinrichtung 4 aufgrund der Lageinformation leicht erzeugt werden.

Ferner weist das Biopsiegerät 3 als weitere Komponente der Ausgabeeinrichtung 5 ein Ausgabemittel 17 auf, vorliegend ein haptisches Ausgabemittel, dass als Vibrationsmotor ausgestaltet ist. So kann unmittelbar und ohne, dass die Person den Blick vom Eingriffsgebiet abwenden muss, eine Ausgabe von Unterstützungshinweisen erfolgen, wie im Folgenden noch genauer erläutert werden wird. Die Unterstützungshinweise werden in Abhängigkeit von der durch die Steuereinheit 13 ermittelten Abweichung von der Sollorientierung ausgegeben.

Die Steuereinheit 13 ist vorliegend als ein Mikrocontroller ausgebildet und weist Firmware auf, um die beschriebenen Funktionen auszuführen.

Um die Komponenten des Biopsiegeräts 3 mit elektrischer Energie versorgen zu können, weist das Biopsiegerät 3 ferner einen aufladbaren elektrischen Energiespeicher 18 auf, beispielsweise eine Batterie. Dank der drahtlosen Kommunikation über die Kommunikationseinrichtung 14 und der integrierten elektrischen Energieversorgung ist das Biopsiegerät 3 kabellos.

Das Biopsiegerät 3 kann, wenn es nicht benötigt wird, in einer Halterung 19 aufbewahrt werden, die beispielsweise an einem Stativ der Bildgebungseinrichtung 2 angeordnet sein kann. Die Halterung ist so ausgebildet, dass das Biopsiegerät 3 nur in einer vorbestimmten Kalibrierungsorientierung in die Halterung 19 eingesetzt werden kann. Immer dann, wenn das Biopsiegerät 3 in der Halterung 19 eingesetzt ist, kann also eine Nachkalibrierung des Lagesensors 12 erfolgen, nachdem die Kalibrierungsorientierung dann bekannt ist. Dass dies der Fall ist, kann beispielsweise dadurch festgestellt werden, dass auch eine Verbindung von Anschlussmitteln 20 des Biopsiegeräts 3 mit Anschlussmitteln 21 einer elektrischen Schnittstelle 22 zum Aufladen des Energiespeichers 18 seitens der Halterung 19 aufgebaut ist, was bei Einsetzen des Biopsiegeräts 3 in die Halterung 19 bevorzugt automatisch geschieht.

Figur 2 zeigt perspektivisch eine Ansicht einer möglichen Ausgestaltung des Biopsiegeräts 3. Die in Figur 1 gezeigten Komponenten des Biopsiegeräts 3 sind in einem Gehäuse 23 aufgenommen, welches Handhabungsmittel 24, beispielsweise Griffe, zur verbesserten Handhabung des Biopsiegeräts 3 aufweisen kann. Wie aus Figur 2 ebenso ersichtlich ist, sind entlang des Nadelschafts der Biopsienadel 8 in regelmäßigen Abständen Markierungen 25 vorgesehen, an denen die Eindringtiefe der Biopsienadel 8 in die Brust 10 abgelesen werden kann, vergleiche auch Pfeil 26 (Figur 1).

Nachdem die Soll-Eindringtiefe auch auf dem Display 16 angezeigt wird, kann somit auch diese durch die die Biopsie durchführende Person leicht eingehalten werden. Auch seitens des Biopsiegeräts 3 können weitere Ausgabemittel vorgesehen sein, um die Soll-Eindringtiefe anzuzeigen.

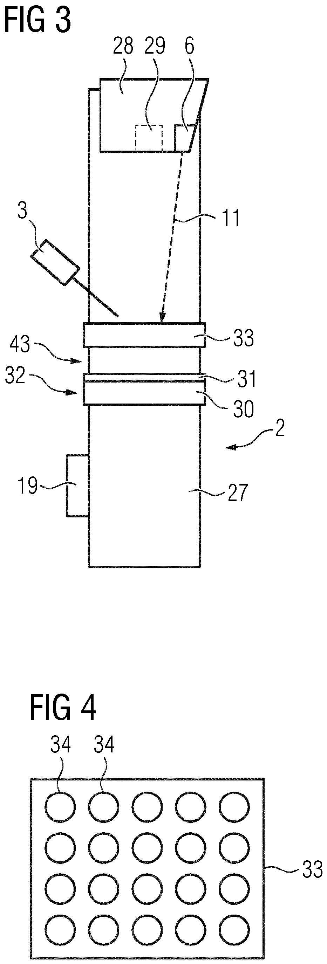

Figur 3 zeigt eine Ansicht der Bildgebungseinrichtung 2, vorliegend eine zur Tomosynthese der Brust 10 ausgebildete Röntgeneinrichtung. Sie umfasst daher ein Stativ 27, an dem eine Strahlereinheit 28 mit dem Röntgenstrahler 29 sowie ein Röntgendetektor 30 mit einer Abdeckung 31 als Objekttisch 32 angeordnet sind. Eine obere, verschiebbare Kompressionsplatte 33 bildet gemeinsam mit dem Detektor 30 und seiner Abdeckung 31 eine Kompressionseinrichtung 43 für die Brust 10.

Damit durch die Kompressionsplatte 33 (oft auch Kompressionspaddle genannt) die Biopsie erfolgen kann, weist diese, wie die Aufsicht in Figur 4 zeigt, Durchgangsöffnungen 34 auf, die vorliegend matrixartig angeordnet sind. Die Planungsinformation kann angeben, welche Durchgangsöffnungen 34 zu nutzen ist. Die Durchgangsöffnungen 34 erlauben es auch, den Eintrittspunkt gemäß dem gestrichelten Pfeil 11 auf die Oberfläche der Brust 10 zu projizieren. Sie erlauben ferner das Einführen der Biopsienadel 8 in die Brust 10 (gestrichelter Pfeil 26).

Für eine konkrete Biopsie gemäß einer Planungsinformation kann nun beispielsweise vorgesehen sein, dass die Steuereinrichtung 4 den Eintrittspunkt an die Markierungseinrichtung 6 überträgt und diese zur Projektion des Eintrittspunkts auf die Oberfläche der Brust 10 ansteuert. Die die Biopsie durchführende Person kann nun das Biopsiegerät 3 in die Hand nehmen und die Biopsienadel 8 auf den Eintrittspunkt aufsetzen bzw. leicht einführen. Er nutzt dann die Unterstützungshinweise der Ausgabeeinrichtung 5, um die Sollorientierung einzunehmen, und im Folgenden die Markierungen 25 der Biopsienadel 8, um die korrekte, der Soll-Eindringtiefe entsprechende Eindringtiefe zu wählen.

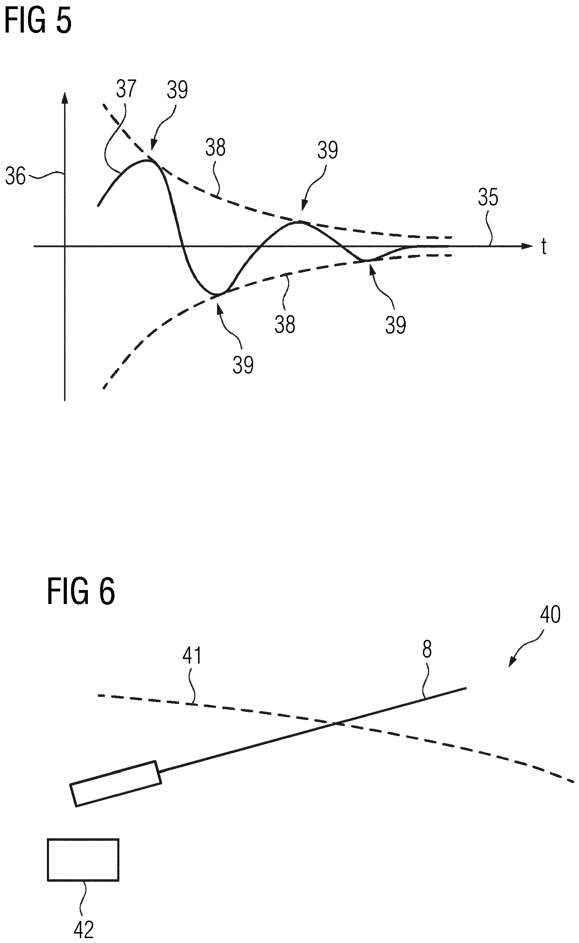

Konkrete Varianten zur Unterstützung der die Biopsie vornehmenden Person bei der Einnahme der Sollorientierung werden bezüglich der Figuren 5 und 6 erläutert. Beide Varianten werden vorzugsweise ergänzend eingesetzt.

Die erste Variante nutzt das haptische Ausgabemittel 17 des Biopsiegeräts 3. Figur 5 zeigt diesbezüglich entlang der Abszisse 35 den Zeitverlauf, entlang der Ordinate 36 die Abweichung der aktuellen Orientierung der Biopsienadel 8 von der Sollorientierung. Der durchgezogene Graph 37 zeigt den zeitlichen Verlauf der von der die Biopsie durchführenden Person vorgenommenen Einstellung, während die gestrichelten Graphen 38 den Verlauf eines sich zeitlich verengenden Zielbereichs um die Sollorientierung (also Abweichung Null) zeigen. Immer dann, wenn die Abweichung an den Rand des Zielbereichs gerät, vergleiche Stellen 39, wird über das Ausgabemittel 17 haptisches Feedback ausgegeben, sodass, wie aus dem Verlauf 37 ersichtlich ist, der Benutzer über die Zeit intuitiv und natürlich, von einer Grobeinstellung zu einer Feineinstellung, an die Sollorientierung geführt wird.

Figur 6 zeigt eine mögliche visuelle, grafische Darstellung 40 auf dem Display 16. Unter Nutzung der Lageinformation kann die Lage der hier stilisiert gezeigten Biopsienadel 8 zu der Sollorientierung 41, gezeigt durch eine gestrichelte Linie, angezeigt werden. Die visuelle Darstellung 40 kann in Echtzeit aktualisiert werden und auch durch weitere Elemente ergänzt werden, insbesondere auch eine hier nur schematisch angedeutete Anzeige 42 der Soll-Eindringtiefe. Die Darstellung 40 kann farblich gestaltet sein.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

Unabhängig vom grammatikalischen Geschlecht eines bestimmten Begriffes sind Personen mit männlicher, weiblicher oder anderer Geschlechteridentität mit umfasst.

## Patentansprüche

1. Biopsieeinrichtung (1) zum Durchführen einer Biopsie an einer weiblichen Brust (10), aufweisend:
- eine Bildgebungseinrichtung (2) zur Tomosynthese der Brust (10) mit einer Kompressionseinrichtung (43) zur Fixierung der Brust (10),
- ein handgehaltenes Biopsiegerät (3) mit einer Biopsienadel, wobei das Biopsiegerät (3) einen Lagesensor (12) zur Messung von die Orientierung der Biopsienadel (8) anzeigenden Lageinformationen aufweist,
- eine mit der Bildgebungseinrichtung (2) registrierte Markierungseinrichtung (6) zur Markierung eines Eintrittspunktes für die Biopsienadel (8) auf der in der Kompressionseinrichtung (43) fixierten Brust (10) gemäß einer auf Bilddaten der Bildgebungseinrichtung (2) basierenden Planungsinformation für eine durchzuführende Biopsie,
- eine Ausgabeeinrichtung (5) zur Ausgabe einer Führungsinformation, die die relative Orientierung der Biopsienadel (8) gemäß der Lageinformation zu einer Sollorientierung (41) gemäß der Planungsinformation anzeigt, für eine die Biopsie durchführende Person, und
- eine Steuereinrichtung (4) zur Steuerung der Biopsieeinrichtung (1) gemäß der Planungsinformation.

2. Biopsieeinrichtung (1) nach Anspruch 1, **dadurch gekennzeichnet, dass** die Markierungseinrichtung (6) einen Markierungslaser (7) aufweist und/oder an der Bildgebungseinrichtung (2) angeordnet ist.

3. Biopsieeinrichtung (1) nach Anspruch 1 oder 2, **dadurch gekennzeichnet, dass** die Kompressionseinrichtung (43) eine obere, insbesondere von einem Röntgendetektor (30) der Bildgebungseinrichtung (2) abgewandt die Brust (10) komprimierende Kompressionsplatte (33) aufweist, die wenigstens eine Durchgangsöffnung (34) zum Durchführen der Biopsienadel (8) aufweist.

4. Biopsieeinrichtung (1) nach einem der vorangehenden Ansprüche, **dadurch gekennzeichnet, dass** sie (1) eine, insbesondere an der Bildgebungseinrichtung (2) angeordnete, Halterung (19) für das Biopsiegerät (3) aufweist.

5. Biopsieeinrichtung (1) nach Anspruch 4, **dadurch gekennzeichnet, dass** die Halterung (19) eine vorbestimmte Kalibrierungsorientierung des Biopsiegeräts (3) zum Kalibrieren des Lagesensors (12) herstellend ausgebildet ist und/oder eine elektrische Schnittstelle (22) zum Aufladen eines elektrischen Energiespeichers (18) des Biopsiegeräts (3) aufweist.

6. Biopsieeinrichtung (1) nach einem der vorangehenden Ansprüche, **dadurch gekennzeichnet, dass** das Biopsiegerät (3) eine Kommunikationseinrichtung (14) zur drahtlosen Kommunikation mit der Steuereinrichtung (4) aufweist.

7. Biopsieeinrichtung (1) nach einem der vorangehenden Ansprüche, **dadurch gekennzeichnet, dass** das Biopsiegerät (3) ein Ausgabemittel (17) der Ausgabeeinrichtung (5) aufweist, welches zur Ausgabe eines Unterstützungshinweises in Abhängigkeit der relativen Orientierung der Biopsienadel (8) gemäß der Lageinformation zu der Sollorientierung (41) ausgebildet ist.

8. Biopsieeinrichtung (1) nach Anspruch 7, **dadurch gekennzeichnet, dass** das Ausgabemittel (17) ein haptisches Ausgabemittel (17) ist.

9. Biopsieeinrichtung (1) nach Anspruch 6 oder 7, **dadurch gekennzeichnet, dass** das Ausgabemittel (17) gemäß einem sich über die Zeit verkleinernden, einzustellenden Zielbereich um die Sollorientierung (41) zur schrittweisen Führung der die Biopsie durchführenden Person zu der Sollorientierung (41) ansteuerbar ist.

10. Biopsieeinrichtung (1) nach Anspruch 9, **dadurch gekennzeichnet, dass** das Ausgabemittel (17) bei Verlassen des Zielbereichs aktiviert wird.

11. Biopsieeinrichtung (1) nach einem der vorangehenden Ansprüche, **dadurch gekennzeichnet, dass** die Ausgabeeinrichtung (5) ein Display (16) umfasst, wobei die Steuereinrichtung (4) zur Ansteuerung des Displays (16) zur Ausgabe einer die relative Orientierung der Biopsienadel (8) gemäß der Lageinformation zu der Sollorientierung (41) visualisierenden Darstellung (40) ansteuert.

12. Biopsieeinrichtung (1) nach einem der vorangehenden Ansprüche, **dadurch gekennzeichnet, dass** sie ferner eine Planungseinrichtung (44) zur wenigstens teilweise automatischen Ermittlung der Planungsinformation aus Bilddaten der Bildgebungseinrichtung (2) aufweist.

13. Biopsieeinrichtung (1) nach einem der vorangehenden Ansprüche, **dadurch gekennzeichnet, dass** die Biopsienadel (8), insbesondere entlang des Nadelschafts, Markierungen (25) zur Anzeige einer Eindringtiefe in eine Patientin aufweist.

14. Verfahren zur Unterstützung einer eine Biopsie mit einer Biopsieeinrichtung (1) nach einem der vorangehenden Ansprüche durchführenden Person, wobei die Ausgabeeinrichtung (5) zur Ausgabe einer Führungsinformation, die die relative Orientierung der Biopsienadel (8) gemäß der Lageinformation zu einer Sollorientierung (41) gemäß der Planungsinformation anzeigt, angesteuert wird.

15. Handgehaltenes Biopsiegerät (3) für eine Biopsieeinrichtung (1) nach einem der Ansprüche 1 bis 13, aufweisend eine Biopsienadel (8) und einen Lagesensor (12) zur Messung von die Orientierung der Biopsienadel (8) anzeigenden Lageinformationen.
